# FASCICULE DE BREVET EUROPEEN

(11) **EP 1 806 396 B1**
(45) Date de publication et mention de la délivrance du brevet: **05.08.2009**
(21) Numéro de dépôt: 06301308.0
(22) Date de dépôt: 29.12.2006
(51) Int. Cl.: C12N 5/06, G01N 33/50

(54) **Modèle de culture cellulaire et ses applications**
Zellkultur-Modell und seine Anwendungen
Cell culture model and its uses

(30) Priorité: 05.01.2006 FR 0650047
(43) Date de publication de la demande: 11.07.2007
(73) Titulaire: L'ORÉAL, 75008 Paris (FR)
(72) Inventeur: Besne, Isabelle, 92800 Puteaux (FR)
(74) Mandataire: Le Coupanec, Pascale A.M.P.

(56) Documents cités:
- WO-A-03/005023
- WO-A-20/05071065
- FR-A- 2 809 412
- GB-A- 2 394 477
- REGNIER M ET AL: "Keratinocyte-melanocyte co-cultures and pigmented reconstructed human epidermis: models to study modulation of melanogenesis" CELLULAR AND MOLECULAR BIOLOGY, CMB ASSOCIATIONS, NOISY-LE-GRAND, FR, vol. 45, no. 7, novembre 1999 (1999-11), pages 969-980, XP009049587 ISSN: 0145-5680
- DOBBIE M ET AL: "Do neuronal cells influence the formation of the blood-brain barrier? Use of a novel tri-culture system" EUROPEAN JOURNAL OF NEUROSCIENCE, vol. 12, no. Supplement 11, 2000, page 355, XP009069548 & MEETING OF THE FEDERATION OF EUROPEAN NEUROSCIENCE SOCIETIES; BRIGHTON, UK; JUNE 24-28, 2000 ISSN: 0953-816X
- CARDINALI GIORGIA ET AL: "Keratinocyte growth factor promotes melanosome transfer to keratinocytes" JOURNAL OF INVESTIGATIVE DERMATOLOGY, vol. 125, no. 6, décembre 2005 (2005-12), pages 1190-1199, XP009069553 ISSN: 0022-202X
- TOYODA, MASAHIKO ET AL: "Calcitonin gene-related peptide upregulates melanogenesis and enhances melanocyte dendricity via induction of keratinocyte -derived melanotrophic factors" JOURNAL OF INVESTIGATIVE DERMATOLOGY SYMPOSIUM PROCEEDINGS , 4(2), 116-125 CODEN: JDSPFO; ISSN: 1087-0024, 1999, XP009069585

## Description

La présente invention a pour objet un modèle multicellulaire comprenant des kératinocytes, des mélanocytes et des cellules nerveuses, ainsi que l'utilisation de ce modèle à des fins de criblages d'agents actifs, notamment à l'égard de la mélanogenèse.

De nombreux modèles cellulaires visant à simuler les différentes propriétés et caractéristiques de la peau ont été mis au point afin de permettre la réalisation des études nécessaires à une meilleure compréhension du rôle des différents éléments constitutifs de la peau, tant dans le domaine mécanique, que dans le domaine physiologique.

De manière générale, ces modèles comprennent des kératinocytes humains déposés sur un support, par exemple un équivalent de derme, et cultivés dans des conditions convenant à la formation d'un équivalent épidermique.

Toutefois, la peau est une structure complexe comprenant divers types cellulaires, le cas échéant, organisés en structures tissulaires, au sein desquelles et entre lesquelles peuvent s'établir des communications cellulaires régulant l'homéostasie de la peau.

A titre d'exemple, une peau comprend, notamment, des kératinocytes, des mélanocytes, des cellules nerveuses, mais également des fibroblastes, des cellules de Langerhans, des cellules endothéliales constituant les vaisseaux sanguins irriguant le derme et l'hypoderme, des cellules musculaires lisses, etc.

Les kératinocytes sont principalement responsables de la production de kératine, un des constituants essentiels de la couche cornée. Ils subissent en permanence une évolution morphologique témoignant de leur kératinisation sous-tendant le rôle de barrière protectrice (mécanique, chimique) de l'épiderme.

Les mélanocytes sont localisés dans la couche basale de l'épiderme. Ils sont le siège de la mélanogenèse, et du fait de leur contact étroit avec les kératinocytes, ils transfèrent à ces derniers la mélanine néo-synthétisée sous la forme de mélanosomes, donnant ainsi à la peau sa coloration.

Le type et la quantité de mélanine contenue dans les mélanosomes déterminent la coloration de la peau. La mélanine constitue, en particulier, un écran efficace de protection contre les rayonnements solaires, notamment les rayonnements ultra-violets.

La mélanogenèse est un phénomène biologique complexe initié par l'hydroxylation de l'acide aminé L-tyrosine résultant de la formation de la L-dihydroxyphénylalanine (L-DOPA), convertie à son tour en DOPA-chrome par l'action d'une enzyme spécifique, associée aux mélanocytes, la tyrosinase. Des réactions de réduction et d'oxydation consécutives conduisent à la conversion du DOPA-chrome en mélanine. La production de la tyrosinase et son activité déterminent en partie la quantité de mélanine produite. La quantité et le type de mélanine transférée aux kératinocytes déterminent, quant à eux, le degré de pigmentation visuelle d'une peau humaine.

Or la mélanine peut être synthétisée de manière excessive, voire anarchique, en réponse à un stress exogène telle que la pollution et les rayons UV, et/ou un stress endogène, par exemple due au vieillissement des kératinocytes, des cellules endothéliales, des fibroblastes et des cellules de Langerhans.

Ainsi, de nombreux troubles cutanés peuvent résulter d'une dérégulation de la mélanogenèse, et conduire, par exemple à une surcharge de mélanine ou à une répartition anormale de la mélanine dans la peau, dénommée hypermélanose. Dans les hypermélanoses, on peut distinguer les mélanodermies, anomalies associées à l'épiderme et les cérulodermies, anomalies dermiques.

Récemment, il a notamment été constaté qu'un stress émotionnel, par exemple d'origine neurogène ou impliquant des cellules nerveuses pouvait induire une libération d'hormones et de neurohormones, capables d'affecter l'homéostasie de la mélanogenèse.

Or à ce jour aucun des modèles cellulaires disponibles ne permet de reproduire et d'étudier l'impact de l'activité nerveuse, de façon satisfaisante, sur les fonctions physiologiques de la peau, et notamment sur la mélanogenèse.

La présente invention vise précisément à satisfaire à ce besoin.

Ainsi, selon un de ses aspects, la présente invention a pour objet un modèle multicellulaire comprenant au moins comme type de cellules :
- des kératinocytes,
- des mélanocytes et
- des cellules nerveuses,
dans lequel les kératinocytes et les mélanocytes forment un premier tapis cellulaire, lesdites cellules nerveuses forment un second tapis cellulaire dénué de contact physique avec le premier tapis cellulaire,
lesdits premier et second tapis cellulaires étant agencés de manière à être compatibles avec une manifestation d'au moins un échange chimique cellulaire.

Le modèle cellulaire selon l'invention a pour avantage d'être aisé à mettre en oeuvre, d'être robuste, et de permettre l'étude des mécanismes physiologiques régulant l'homéostasie de la peau, et notamment de la mélanogenèse.

Le modèle cellulaire selon l'invention a également pour avantage d'être simple à mettre en oeuvre, de ne pas nécessiter de contact direct entre les cellules nerveuses et les autres cellules, en particulier avec les co-cultures de mélanocytes et de kératinocytes.

Ainsi, les inventeurs ont observé que l'application de noradrénaline dans un modèle multicellulaire selon l'invention entraîne une augmentation de la libération de Calcitonine-Gene-Related Peptide (CGRP) à partir de terminaisons de cellules nerveuses, ainsi qu'une augmentation de la synthèse de mélanine par des mélanocytes. En revanche l'administration d'un antagoniste de récepteurs au CGRP, le CGRP₈₋₃₇, se traduit, plus particulièrement, par une diminution importante de la synthèse de mélanine, témoignant ainsi de l'implication du CGRP dans un échange chimique cellulaire entre les cellules nerveuses et les mélanocytes.

Selon un autre de ces aspects, la présente invention a pour objet une utilisation d'un modèle multicellulaire conforme à l'invention pour cribler des agents susceptibles de moduler une manifestation d'un échange chimique susceptible d'intervenir entre des cellules nerveuses, des kératinocytes et/ou des mélanocytes.

Selon encore un autre de ces aspects, la présente invention a pour objet un procédé de criblage d'agents susceptibles de moduler la mélanogenèse, comprenant au moins les étapes consistant à :
a) disposer d'un modèle multicellulaire conforme à l'invention dans des conditions propices à la manifestation de la mélanogenèse,
b) mettre en présence dudit modèle, et dans des conditions propices à une interaction avec ledit modèle, au moins un agent à cribler, et
c) déterminer la quantité de mélanine produite par ledit modèle, à l'issue de l'étape b).

Ainsi, le modèle selon l'invention a pour avantage de permettre de déterminer de nouvelles cibles biologiques susceptibles d'être impliquées dans des désordres cutanés résultant d'une altération de la mélanogenèse.

De même un modèle multicellulaire selon l'invention peut également s'avérer utile pour cribler des agents susceptibles de moduler la mélanogenèse et d'identifier de nouveaux agents thérapeutiques et/ou cosmétiques efficaces pour la dépigmentation ou la propigmentation.

Au sens de l'invention, « multicellulaire » signifie au moins trois types cellulaires distincts.

Au sens de la présente invention, on entend désigner par l'expression "tapis cellulaire", un ensemble de cellules, à confluence ou non, en monocouche ou en multicouche, disposé dans un même plan.

Au sens de l'invention, on entend désigner par l'expression "échange chimique cellulaire", l'ensemble des signaux figurés par des molécules, libérées à partir d'une cellule, et susceptible d'affecter, à distance, l'activité d'une autre cellule, appartenant ou non au même type cellulaire. Une telle molécule peut être, par exemple et de manière non limitative, un peptide, une protéine, un lipide, un sucre, une hormone stéroïdienne, une catécholamine. Elle peut être libérée sous forme d'une sécrétion, comme par exemple la libération de molécules de CGRP.

Au sens de la présente invention, l'expression « dénué de contact physique » vise à désigner un agencement dans lequel le premier et le second tapis cellulaires, disposés ou non dans une unique enceinte, sont mis en relation l'un avec l'autre au moyen du milieu de culture dans lequel ils sont incubés, sans que des cellules d'un tapis puissent entrer, directement, en contact avec des cellules d'un autre tapis, par exemple par contact entre les corps cellulaires ou au moyen de prolongements cellulaires, telles que des axones ou dendrites. L'expression « dénué de contact physique » signifie notamment que les cellules nerveuses ne font pas de contact synaptique avec les autres types de cellules présents dans un modèle de l'invention.

Ainsi, dans un modèle multicellulaire selon l'invention, les cellules nerveuses du second tapis cellulaire n'innervent pas les cellules du premier tapis cellulaire.

Au sens de l'invention, on entend par le terme « moduler », désigner une augmentation ou une diminution d'une activité biologique d'une cellule donnée, par exemple la mélanogenèse.

### MODELE MULTICELLULAIRE

Le modèle multicellulaire selon l'invention comprend un premier tapis cellulaire comprenant des kératinocytes et des mélanocytes, et un second tapis cellulaire comprenant des cellules nerveuses.

Les premier et second tapis cellulaires sont dénués de contact physique entre eux, et sont agencés de manière à être compatibles avec la manifestation d'au moins un échange chimique cellulaire.

A titre d'exemple d'enceinte convenant à la mise en oeuvre de l'invention, il peut être mentionné des puits de plaques de culture telles que des plaques de culture cellulaire de 6, 12, 24, 48 puits ou de 96 puits, usuellement utilisées en culture de cellules.

Selon un mode de réalisation, le premier et/ou le second tapis cellulaire peut (peuvent) être disposé(s) sur et/ou dans un support poreux ou semi-perméable.

Par support poreux, on entend en particulier un insert dont la base comprend des pores.

La taille des pores sera adaptée par l'homme du métier de manière à permettre, éventuellement, le développement de prolongements cellulaires sans que ne s'établissent de contacts directs entre le premier et le second tapis cellulaires. Par exemple, la taille des pores pourra varier de 0,001 à 10 µm, de préférence supérieure ou égale à 0,5 µm. A titre d'exemple non limitatif, la base de l'insert poreux convenant à l'invention, pourra ainsi comprendre une matrice poreuse de collagène, comprenant optionnellement des glycosaminoglycanes et/ou des fibroblastes, un gel ou une membrane d'acide hyaluronique et/ou de collagène et/ou de fibronectine et/ou de fibrine, une membrane semi-perméable de nitrocellulose, de nylon^{®}, de téflon^{®}, de polycarbonate ou de polyéthylène ou de polypropylène ou de polyethylène térephtalate (PET), une membrane inorganique Anopore^{®} semi-perméable, une membrane d'acétate de cellulose, une membrane semi-perméable Biopore-CM^{®}, une membrane semi-perméable de polyester et une membrane d'acide polyglycolique.

Par exemple, il est possible de former deux plans de tapis cellulaires en cultivant, un premier ensemble de cellules en insert de culture, puis en disposant ce ou ces insert(s) dans des plaques de culture dont le fond comprend un second ensemble de cellules formant un second tapis cellulaire.

Selon un mode de réalisation, un support poreux convenant à la mise en oeuvre de la présente invention peut être un insert agencé de manière à être disposé dans un puits de plaque de culture cellulaire sans être en contact direct avec le fond dudit puits.

Ainsi, un tel support peut comporter des ergots, des crochets ou tout autre moyen permettant son maintien à une distance appropriée du fond d'un puits de plaque de culture cellulaire et notamment du tapis cellulaire qui y est éventuellement disposé.

A titre d'exemple d'insert convenant à la mise en oeuvre de l'invention, on peut mentionner, de manière non exhaustive, l'insert à membrane PET (polyéthylène téréphtalate) transparentes ou opaques de marque Falcon^{®}, Nunc^{®} ou Costar^{®}. Des inserts convenant particulièrement à la mise en oeuvre de l'invention, sont également les inserts commercialisés sous la référence THINCERT et dont la porosité est de 1 µm (Réf : 662610) par la société GREINER BIO-ONE.

Les premier et second tapis cellulaires peuvent être agencés de manière à diviser une enceinte unique en au moins deux compartiments.

Selon un mode de réalisation, lesdits premier et second tapis cellulaires peuvent être agencés selon des plans parallèles.

Selon un mode de réalisation, le premier et le second tapis cellulaires peuvent être agencés selon des plans parallèles horizontaux, et notamment dans lesquels le second tapis figure un plan supérieur.

Selon un mode de réalisation, le plan supérieur peut être formé par un fond d'un insert dans lequel est disposé le second tapis cellulaire.

Avantageusement, le fond de l'insert peut présenter une porosité convenant au développement de terminaisons nerveuses à l'extérieur dudit fond, pour favoriser l'échange chimique attendu, sans toutefois qu'un contact direct, notamment par innervation, ne s'établisse entre les tapis cellulaires.

Un tel insert approprié peut être choisi parmi les supports poreux définis précédemment.

Selon ce mode de réalisation, le premier tapis cellulaire est pour sa part disposé au fond de l'enceinte, optionnellement sur un support plus particulièrement choisi parmi une matrice de collagène, comprenant éventuellement des fibroblastes et/ou des glycosaminoglycanes, un derme désépidermisé, un équivalent de derme, une membrane d'acide hyaluronique et/ou de collagène et/ou de fibronectine et/ou de fibrine, et un support inerte.

Selon un mode de réalisation, le modèle multicellulaire conforme à l'invention peut comprendre, en outre, au moins un type cellulaire additionnel, par exemple, choisi parmi des cellules endothéliales, des cellules du système immunitaire telles que des cellules de Langerhans, des lymphocytes T, des cellules dendritiques, des macrophages, ou encore des adipocytes. Le(les)dit(s) type(s) cellulaire(s) additionnel(s) peuvent être co-cultivé(s) avec le premier et/ou le second tapis cellulaire.

Un exemple de réalisation d'un modèle multicellulaire conforme à l'invention peut être obtenu comme suit.

Une co-culture de kératinocytes et de mélanocytes peut être effectuée selon des conditions de culture cellulaire standard, directement sur le fond d'une plaque de culture.

Cette co-culture est généralement initiée par un ensemencement des puits par soit des mélanocytes, soit des kératinocytes, notamment tels que définis dans les exemples ci-après. Après une période de temps convenant à l'adhésion des mélanocytes ou kératinocytes ensemencés, et allant généralement de quelques heures à quelques jours, notamment comprise entre 1 heure et 72 heures, le second type de cellules, kératinocytes ou mélanocytes, selon le cas, peut être ensemencé dans les puits contenant le premier type de cellules préalablement introduit.

Les densités cellulaires à mettre en oeuvre pour l'ensemencement des plaques de culture par les kératinocytes et les mélanocytes peuvent être ajustées selon différents facteurs, tels que le type de cellules, la taille des cellules, la vitesse de prolifération des cellules, la surface des puits. Un tel ajustement relève de la pratique de routine de l'homme de l'art.

Des cellules nerveuses peuvent être cultivées sur un support poreux, tel que par exemple des inserts THINCERT mentionnés précédemment.

A titre d'exemple de cellules nerveuses convenant à la mise en oeuvre de l'invention, on peut mentionner les cellules nerveuses sensitives animales ou humaines présentes au niveau de la peau et capables de libérer des neuromédiateurs, ou des cellules nerveuses capables de libérer tout facteur capable de moduler la mélanogenèse ... Il peut également s'agir de lignées cellulaires neuronales humaines (originales ou métastasiques) qui présentent les caractéristiques des cellules nerveuses avec libération de neurohormones ou neurotransmetteurs.

Après une période de culture adéquate, généralement 2 à 6 jours, les cellules nerveuses, disposées dans et/ou sur un insert ou dans et/ou sur un support poreux, peuvent être introduites dans un puits de plaque de culture de cellules comprenant une co-culture de kératinocytes et de mélanocytes.

L'ensemble des cellules d'un modèle multicellulaire conforme à l'invention peut être mis en culture dans un milieu adapté au maintien et/ou à la croissance et/ou à la prolifération de chacun des types cellulaires du modèle multicellulaire conforme à l'invention.

De nombreux milieux de culture susceptibles de convenir à la mise en oeuvre de l'invention peuvent être obtenus commercialement. A titre d'exemples, non exhaustifs, de milieux de culture convenant à l'invention, on peut mentionner le Dulbecco's Modified Eagle's Medium (DMEM), le Minimal Essential Medium (MEM), le M199, le RPMI 16-40 ou l'Iscove's Modified Dulbecco's Medium (EDMEM), le Ham's F-12, le Ham's F-10, le NCTC 109 et le NCTC 135.

Ces milieux peuvent être complémenter par tout additif classiquement utilisé en culture cellulaire tel que, par exemple et de manière non limitative, des précurseurs de phospholipides, des acides aminés non essentiels, des acides nucléiques, des vitamines, des antibiotiques, des co-facteurs enzymatiques, des sels minéraux, de l'insuline, de la transferrine, de la triiodothyronine, de l'éthanolamine, de l'o-phosphoryl-éthanolamine ou des facteurs de croissance tels que le facteur de croissance nerveuse ou la neurotrophine-3.

Les concentrations des différents additifs usuellement utilisés pour complémenter les milieux de culture cellulaire peuvent être déterminer et adapter par l'homme de l'art, notamment selon le type de cellules à cultiver.

D'autres milieux sont décrits dans HAM and McKEEHAN, « Methods in Enzymology », 58:44-93, 1979, ou encore dans BOTTENSTEIN et al., « Methods in Enzymology », 58:94-109, 1979.

Par ailleurs, il est également possible d'utiliser des mélanges de différents milieux notamment des milieux précités, tels que par exemple un mélange de DMEM/HAM F12.

A titre d'exemple, non limitatif, de milieu de culture convenant à la mise en oeuvre de l'invention, il est possible de mentionner un milieu de co-culture comprenant un mélange de milieux DMEM-HAM F12 commercialisés par la société INVITROGEN sous la référence 21331-020, et de milieux M154 commercialisés par la société TEBU sous la référence M154 CF/PRF.

Selon un mode de réalisation, un modèle multicellulaire de l'invention est en particulier mis en oeuvre dans un milieu de culture tel que défini dans les exemples ci-après illustrant l'invention.

Ce milieu peut en outre être complémenté avec un ou plusieurs des additifs classiquement utilisés en culture de cellules, tels que par exemple de la L-glutamine, des antibiotiques tels que la pénicilline, la streptomycine, des facteurs de croissance tels que le facteur de croissance nerveuse (*nerf growth factor,* NGF), de la neurotrophine-3 (NT-3), un facteur de croissance des kératinocytes humaines (*human keratinocyte growth supplement,* HKGS), ou des sels minéraux tels que le chlorure de calcium (CaCl₂).

De manière générale, un modèle multicellulaire conforme à la présente invention peut être maintenu dans des conditions de culture de maintien de survie et/ou de croissance cellulaire et/ou de prolifération cellulaire pendant une durée variant d'environ 5 à 15 jours, dans des conditions de culture standard.

Dans ces conditions, les mélanocytes peuvent effectuer une mélanogénèse représentative d'un état basal physiologique.

### MISE EN OEUVRE DU MODELE MULTICELLULAIRE

Il convient de noter qu'un modèle cellulaire conforme à l'invention n'est pas limité à la seule mise en oeuvre de l'étude de l'influence des cellules nerveuses sur la mélanogenèse. Il relève de la pratique de l'homme de l'art d'envisager de nombreuses variantes de l'invention sans sortir du cadre de celle-ci.

Ainsi, il peut également être envisagé, par exemple et de manière non limitative, de mettre en oeuvre un modèle conforme à l'invention pour l'étude de l'influence de l'activité des cellules nerveuses sur les kératinocytes, ou l'influence de l'activité de mélanocytes sur l'activité des cellules nerveuses ou des kératinocytes, etc.

A titre d'exemple d'échange chimique cellulaire considéré dans un modèle multicellulaire conforme à l'invention, on peut aussi mentionner l'ensemble des signaux extracellulaires découlant de l'application sur des cellules nerveuses d'un neurotransmetteur de type adrénergique, tel que la noradrénaline (NA), ou cholinergique tel que l'acétylcholine, ou des agonistes mimant l'activité de ces substances tels que, respectivement, l'isoprotérénol ou la nicotine ou muscarine.

L'échange chimique cellulaire considéré peut avoir un effet modulateur à l'égard d'une activité biologique d'au moins un type de cellule.

A titre d'exemple d'activité biologique susceptible d'être modulée par un échange chimique cellulaire considéré dans un modèle multicellulaire selon l'invention, on peut mentionner la mélanogenèse ou encore la libération de neurotransmetteurs.

Selon un mode de réalisation, une activité biologique peut être la mélanogenèse.

Une modulation de la mélanogenèse peut résulter d'un échange chimique cellulaire susceptible de s'effectuer directement entre des cellules nerveuses et des mélanocytes, ou éventuellement en comprenant un stade intermédiaire au niveau des kératinocytes.

Selon un mode de réalisation, l'échange chimique susceptible d'être modulé par un agent criblé peut avoir un effet modulateur à l'égard de la mélanogenèse.

Cette mise en oeuvre de cet aspect de l'invention peut être effectuée selon un procédé de criblage d'agents comprenant au moins les étapes consistant à :
a) disposer d'un modèle multicellulaire conforme à l'invention,
b) mettre en présence dudit modèle multicellulaire, au moins un agent à cribler, et
c) déterminer la quantité de mélanine produite par ledit modèle à l'issue de l'étape b).

Avantageusement, le modèle multicellulaire selon l'invention présente des conditions propices à la manifestation de la mélanogenèse.

Selon un mode particulier de réalisation, la quantité de mélanine produite à l'issue de l'étape c) peut être comparée à une quantité de mélanine produite par un modèle conforme à l'invention en absence d'agent à cribler et/ou en présence d'une molécule de référence dont l'effet sur la mélanogenèse est connu, par exemple un inhibiteur de la mélanogenèse tel que le CGRP₈₋₃₇, ou par exemple un activateur de la mélanogenèse comme la noradrénaline.

L'agent à cribler est mis en présence du modèle cellulaire à l'étape b) et dans des conditions propices à une interaction avec ledit modèle, en particulier par contact avec au moins un des types de cellules en culture et/ou au moins un milieu de culture desdites cellules, notamment dans un insert et/ou dans un puits.

Selon un mode particulier de réalisation, l'agent à cribler peut être susceptible de moduler la mélanogenèse par action au niveau des cellules nerveuses et/ou des mélanocytes et/ou des kératinocytes.

Un agent à cribler peut être par exemple un inhibiteur de la mélanogenèse agissant directement sur les mélanocytes, un inhibiteur de la mélanogenèse agissant sur les cellules nerveuses, un inhibiteur de la mélanogenèse agissant sur les kératinocytes, un agent stimulateur de la mélanogenèse agissant directement sur les mélanocytes, un agent stimulateur de la mélanogenèse agissant sur les kératinocytes, ou un agent stimulateur de la mélanogenèse agissant sur les cellules nerveuses, par exemple en stimulant la libération d'une molécule susceptible d'activer, en retour, la mélanogenèse au niveau des mélanocytes.

Selon un mode particulier de réalisation, la mélanogenèse peut être stimulée par la mise en oeuvre dans l'étape a) d'un agent activant ou inhibant un type de cellules.

En particulier, un tel agent peut stimuler les cellules nerveuses, et augmenter ainsi la libération de médiateurs chimiques.

Plus particulièrement, l'agent peut être choisi parmi des agonistes adrénergiques tel que la noradrénaline ou l'adrénaline.

En effet, il a été observé, dans le cadre de l'invention, que la stimulation de la mélanogénèse par la noradrénaline ne s'effectue pas seulement par stimulation directe, mais peut faire intervenir un facteur intermédiaire libéré par les terminaisons nerveuses.

Ainsi, il est par exemple possible de stimuler l'activité des cellules nerveuses par mise en contact de ces dernières avec un agent stimulant leur activité, tel qu'un agoniste noradrénergique, comme par exemple la noradrénaline. Cette activation des cellules nerveuses peut alors résulter en une libération de neurohormones, comme par exemple le CGRP, qui peut à son tour agir sur les mélanocytes en stimulant la mélanogenèse.

Un agent à cribler peut être mis en contact avec un modèle multicellulaire selon l'invention avant, après ou simultanément avec l'addition d'un agent activateur ou inhibiteur d'un type de cellule.

La mise en présence d'un agent activateur ou inhibiteur d'un type de cellules et d'un agent à cribler en étape b) peut être effectuée pendant un temps adéquat pour permettre de déterminer une variation possible de la quantité de mélanine.

Par exemple, le temps d'incubation de noradrénaline en absence ou en présence d'un agent à cribler peut être d'environ 5 à 12 jours, et notamment d'environ 10 jours.

Selon un mode de réalisation, la quantité de mélanine produite par un modèle selon l'invention, en absence d'agent à cribler et d'agent activateur ou inhibiteur d'un type de cellules, peut être comparée à une quantité de mélanine obtenue en présence d'un agent activateur ou inhibiteur, mais en absence d'un agent à cribler, et à une quantité de mélanine obtenue en présence de ces deux types d'agents.

Ces différentes quantités de mélanine peuvent être obtenues en parallèle dans une même série d'expériences.

La quantité de mélanine produite par les mélanocytes peut être déterminée, par exemple, par mesure de la densité optique, par exemple à 405 nm, après extraction de cette dernière par toutes techniques appropriées et connues de l'homme de l'art.

Par exemple, il est possible d'extraire la mélanine au moyen d'une solution de soude (NaOH à 0,5 N).

Les valeurs de densité optique mesurées peuvent être ensuite comparées à une gamme de mélanine exogène, par exemple variant d'environ 0,3 à environ 100 µg/ml de mélanine.

Un modèle multicellulaire conforme à l'invention peut également être utilisé pour identifier au moins une molécule susceptible d'être impliquée dans une manifestation d'un échange chimique cellulaire susceptible d'intervenir entre des cellules nerveuses, des kératinocytes et/ou des mélanocytes.

Selon un mode de réalisation, l'échange chimique considéré peut avoir un effet modulateur à l'égard de la mélanogenèse.

Ainsi, un modèle multicellulaire selon l'invention peut être mis en oeuvre, par exemple, à des fins d'identification de molécules susceptibles d'être libérées par des cellules nerveuses et susceptibles d'agir sur des mélanocytes pour moduler la mélanogenèse.

L'identification de telles molécules peut être effectuée par toutes techniques appropriées connues de l'homme de l'art, par exemple à partir d'un échantillon de milieu de culture prélevé à partir d'un modèle cellulaire conforme à l'invention.

Les moyens d'identification susceptibles d'être mis en oeuvre peuvent être par exemple un dosage ELISA, une méthode de séparation analytique par chromatographie, par exemple une chromatographie liquide à haute performance, éventuellement couplée à un spectromètre de masse, une RMN ou un spectromètre infrarouge.

De nombreuses modifications de l'invention telle qu'exposée ci-dessus peuvent être envisagées par l'homme de l'art sans s'écarter de la portée de celle-ci.

De telles modifications sont couvertes par la présente demande.

L'invention est illustrée par les exemples suivants, qui ne doivent pas être interprétés comme limitant la portée de la présente invention.

### FIGURES ET LEGENDES

Figure 1 : Représentation schématique d'un modèle multicellulaire de tri-culture kératinocytes/mélanocytes/cellules nerveuses.

L'insert est introduit dans le puits de culture de telle sorte que les kératinocytes, les mélanocytes et les cellules nerveuses sont cultivés dans le même milieu de culture afin de permettre des échanges chimiques intracellulaires.

Figure 2 : Représente la croissance des terminaisons nerveuses des cellules nerveuses d'un modèle multicellulaire cellule nerveuse-kératinocytes-mélanocytes à travers l'insert poreux sur lequel elles sont cultivées.

Les photographies ont été prises par microscopie à épifluorescence après marquage des cellules nerveuses avec un anticorps tubuline et révélation avec un anticorps anti-immunoglobuline marqué par Alexa Fluor 488. Elles ont été prises en-dessous de l'insert (côté kératinocytes-neurones).

Les flèches indiquent les prolongements ayant traversé le support et se développant côté puit de culture.

La barre blanche représente 5 µm.

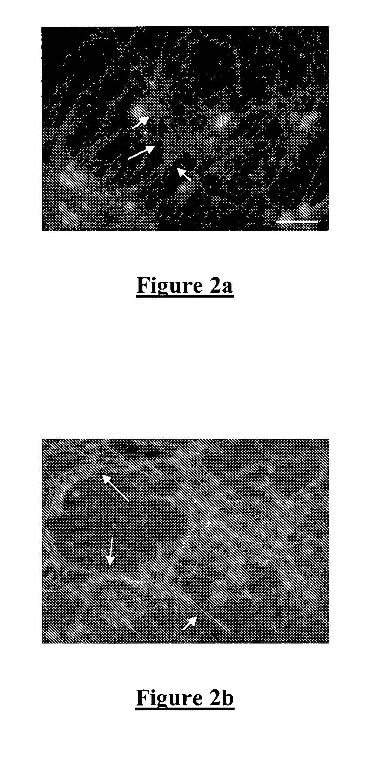

La figure 2a représente des cellules nerveuses cultivées en absence de noradrénaline, et la figure 2b représente des cellules nerveuses cultivées en présence de noradrénaline à raison de 10⁻⁵M.

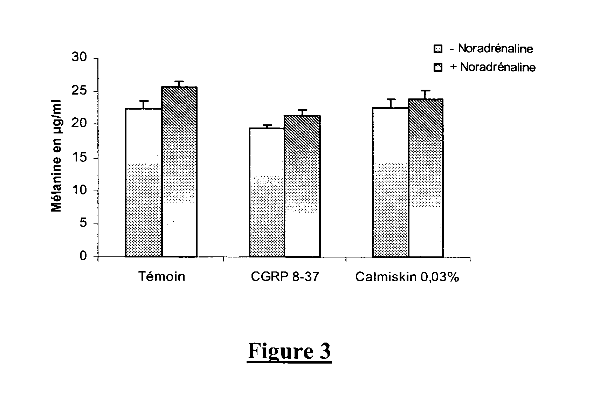

Figure 3 : Représente la modulation de la mélanogenèse par l'activité des cellules nerveuses. Les cellules nerveuses d'un modèle multicellulaire selon l'invention sont stimulées, ou non, par de la noradrénaline pendant 10 jours, et le cas échéant en présence de CGRP₈₋₃₇ ou de CALMISKIN^{®} à 0,03 %.

### EXEMPLES

### Exemple 1

### Préparation d'un modèle multicellulaire de kératinocytes/mélanocytes/cellules nerveuses.

Une première culture a été préparée à partir de neurones sensitifs (N) cultivés en inserts de culture pour plaque 24 puits (ThinCert^{™}, Greiner bio-one ref. 662 610) dans un milieu de culture DMEM-HAM F12 (Invitrogen 21331-020) complémentés avec de la L-glutamine 2mM (Invitrogen 25030024), de la pénicilline 50 UI/ml - Streptomycine 50 µg/ml (Invitrogen 15070063), un supplément N2 (17502-048), un facteur de croissance nerveux (Nerf Growth Factor, NGF, Invitrogen 13290.010) et de la neurotrophine 3 (NT-3, Tebu 450-03-b).

Ces inserts ont une surface de 0,33 cm² et une porosité de 1 µm. Ils ont également la propriété d'être transparents, ce qui permet d'observer les cellules.

20 000 cellules neuronales ont été ensemencées par insert en milieu défini pour la culture de neurones sensitifs dans une étuve à 37 °C et 5 % CO₂ saturée en humidité.

Les neurones ont été maintenus en culture pendant 4 jours dans un incubateur maintenu à 37 °C, atmosphère de 5 % de CO₂.

Une seconde culture a été préparée avec des kératinocytes (K) (kératinocytes humains normaux (NHEK) isolés à partir de chirurgie plastique et utilisés au 3^{ème} passage) ensemencés à raison de 100 000 cellules/puits, en plaque 24 puits, en milieu (Médium 254 (Tebu 058M-254-500) complémenté avec HMGS-2 sans PMA (Tebu 058S-016-5) et un mélange de pénicilline 50 UI/ml et de streptomycine 50 µg/ml.

Après 3 heures d'adhésion, des mélanocytes (M) (mélanocytes épidermiques humains normaux (NHEM-2) utilisés au 6^{ème} passage) ont été ensemencés à raison de 50 000 cellules/puits dans les cultures de kératinocytes.

Les kératinocytes-mélanocytes ont été maintenus en culture pendant 1 jour, dans un incubateur maintenu à 37 °C, atmosphère de 5 % de CO₂.

Après 4 jours de culture, les inserts contenant les cellules nerveuses ont été déposées dans les puits contenant la co-culture kératinocytes/mélanocytes (après 1 jour de culture de co-culture), pour obtenir le modèle multicellulaire cellules nerveuses-kératinocytes-mélanocytes (N/K/M), en présence de milieu de culture N/K/M.

Le milieu de culture N/K/M comprend un mélange 50-50 de milieu DMEM-HAM F12 (Invitrogen 21331-020) complémenté comme indiqué précédemment, pénicilline 50 UI/ml, de la streptomycine 50 µg/ml (Invitrogen 15070063), un supplément et de milieu M 154 (Tebu M 154 CF/PRF) complémenté avec du CaCl₂ (Tebu S-013-154) et un facteur de croissance pour kératinocytes (Complement Human Keratinocytes Growth supplement HKGS Tebu S-001-5).

Le modèle multicellulaire ainsi obtenu a été maintenu en culture pendant au moins 10 jours.

Le milieu de culture du puits et de l'insert a été renouvelé pour moitié chaque jour.

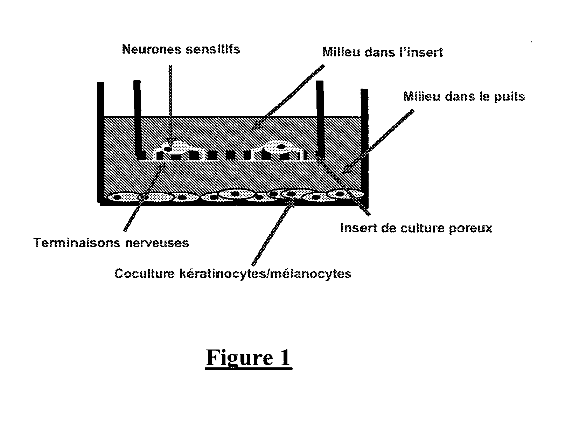

Un schéma illustrant le modèle multicellulaire ainsi obtenu est représenté Figure 1.

### Exemple 2

### Mise en évidence de la croissance des terminaisons nerveuses à travers un support poreux

A partir d'un modèle multicellulaire tel qu'obtenu à l'exemple 1, les cellules ont été cultivées en présence, ou non, de noradrénaline 10⁻⁵M, pendant 10 jours.

Le milieu de culture N/K/M a été déposé dans les puits de culture et dans les inserts.

Les différents milieux ont été renouvelés pour moitié tous les jours.

Les cellules nerveuses ont été marquées avec un anticorps monoclonal anti-β-tubuline (Sigma T8660), puis révélées par un conjugué anticorps de chèvre anti-immunoglobuline de souris-Alexa fluor 488 (Interchim A-11029).

Après lavage extensif en PBS, les préparations ont été observées en épifluorescence (microscope Nikon Diaphot 300) et photographiées.

Les images sont présentées à la figure 2.

Les photographies ont été prises en-dessous de l'insert (côté kératinocytes-neurones).

Les flèches indiquent les prolongements ayant traversé le support et se développant côté puit de culture.

La barre blanche représente 5 µm.

La figure 2a représente des cellules nerveuses cultivées en absence de noradrénaline, et la figure 2b représente des cellules nerveuses cultivées en présence de noradrénaline à raison de 10⁻⁵M.

On constate qu'en présence de noradrénaline, la densité des prolongements cellulaires est augmentée.

### Exemple 3

### Effets d'un stress émotionnel de type noradrénergique sur la mélanogenèse

Des cellules d'un modèle multicellulaire tel qu'obtenu à l'exemple 1 ont été maintenues pendant 10 jours en présence ou non de noradrénaline 10⁻⁵M, additionnée ou non de CALMISKIN^{®} 0,03 % ou de CGRP₈₋₃₇ 10⁻⁶M , selon un protocole similaire à celui décrit à l'exemple 2.

Le CGRP₈₋₃₇ est un antagoniste de référence des récepteurs au CGRP.

Le produit CALMISKIN^{®} est une solution d'extrait de feuille de menthe (mentha piperita) à 100 % (v/v) dans l'eau et est distribuée par la société SILAB.

A l'issue de l'incubation, la mélanine été extraite des co-cultures de kératinocytes/mélanocytes par une solution de NaOH 0,5 N, puis dosée par mesure de la densité optique (405 nm) et comparée à une gamme étalon de mélanine exogène (0,39-100 µg/ml de mélanine Sigma M8631).

Les données expérimentales ont été analysées au moyen du logiciel PRISM^{®} (Graph Pad Software).

Les comparaisons intergroupes ont été réalisées par analyse de variance (ANOVA) à l'aide du test de comparaison multiple de Dunnet.

Les comparaisons entre deux échantillons ont été réalisées par analyse à l'aide du T test.

Les résultats obtenus sont présentés dans le tableau ci-dessous. Ils représentent la moyenne de 4 expériences indépendantes. Ils sont exprimés en µg de mélanine/ml.

| | **sans Noradrénaline** | **+ Noradrénaline** |
|---|---|---|
| Témoin | 22,4 ± 1,12 | 25,77 ± 0,72^{#} |
| CGRP₈₋₃₇ 10⁻⁶M | 19,33 ± 0,48 | 21,35 ± 0,91^{#} |
| CALMISKIN 0,03 % | 22,57 ± 1,30 | 23,89 ± 1,32* |

La présence de Noradrénaline 10⁻⁵M stimule de façon significative (#; p<0,01) la quantité de mélanine synthétisé dans les cocultures (+ 15 %).

Le produit CALMISKIN à la concentration de 0,03 %, ainsi que l'antagoniste des récepteurs de la CGRP, le CGRP₈₋₃₇ diminuent de façon significative (*; p<0,05 et # ; p<0,01) la quantité de mélanine (- 7,3 % et -17,2 % respectivement) en présence de noradrénaline.

### Exemple 4

### Mesure de la libération de CGRP

A l'issue de l'expérience décrite à l'exemple 3, les surnageants des puits et des inserts ont été recueillis séparément et congelés immédiatement à -80 °C afin d'analyser le contenu en CGRP.

Le contenu en CGRP a été mesuré dans les surnageants de culture par un test ELISA (Rat CGRP enzyme Immuno Assay Kit, Spi Bio A05482) selon le protocole préconisé par le fournisseur.

Les résultats sont exprimés en pg de CGRP/ml de milieu.

L'analyse des résultats a été effectuée comme décrit précédemment.

| | **Puits de culture** | **Insert** |
|---|---|---|
| - Noradrénaline | 159,31 ± 21,24 | 417,12 ± 37,33 |
| + Noradrénaline 10⁻⁵ M | 170,48 ± 19,47 | 493,86 ± 11,57 |
| Noradrénaline 10⁻⁵ M + CALMISKIN 0,03 % | 134,11 ± 14,66* | 474,19 ± 12,46 |

| | | |
|---|---|---|
| *p<0,05 | | |

En présence de noradrénaline, la quantité de CGRP libérée dans 1' insert de la culture témoin est très importante. Le produit CALMISKIN^{®} a modulé légèrement la libération de CGRP.

La quantité de CGRP dosée dans le milieu de culture des puits (au contact de la coculture de kératinocytes/mélanocytes) montre que le CGRP a diffusé ou a été libéré par les terminaisons nerveuses ayant traversées l'insert poreux. A ce niveau, le produit CALMISKIN a diminué significativement la libération de CGRP (- 21 % du témoin ; p<0,05).

### Conclusion

L'addition de noradrénaline à un modèle multicellulaire selon l'invention induit une augmentation de la quantité de CGRP libérée dans le milieu de culture par les cellules nerveuses.

Cette quantité est plus importante au niveau des cellules nerveuses mais est également mesurable à proximité de la coculture kératinocytes/mélanocytes.

La noradrénaline apparaît, ainsi, augmenter la mélanogenèse de façon mesurable (+ 15 %), au niveau de la coculture kératinocytes/mélanocytes par le biais de la libération de CGRP.

Cette stimulation a été diminuée par application du produit CALMISKIN à la concentration de 0,03 %.

## Revendications

1. Modèle multicellulaire comprenant au moins comme type de cellules :
- des kératinocytes,
- des mélanocytes, et
- des cellules nerveuses,
dans lequel les kératinocytes et les mélanocytes forment un premier tapis cellulaire, lesdites cellules nerveuses forment un second tapis cellulaire dénué de contact physique avec le premier tapis cellulaire,
lesdits premier et second tapis cellulaires étant agencés de manière à être compatibles avec une manifestation d'au moins un échange chimique cellulaire.

2. Modèle multicellulaire selon la revendication précédente, dans lequel l'échange chimique cellulaire a un effet modulateur à l'égard d'une activité biologique d'au moins un type de cellule.

3. Modèle multicellulaire selon la revendication précédente, dans lequel l'activité biologique est la mélanogenèse.

4. Modèle multicellulaire selon l'une quelconque des revendications précédentes, dans lequel lesdits premier et second tapis cellulaires sont agencés de manière à diviser une enceinte unique en au moins deux compartiments.

5. Modèle multicellulaire selon l'une quelconque des revendications précédentes, dans lequel le premier et/ou le second tapis cellulaire est (sont) disposé(s) sur et/ou dans un insert poreux ou semi-perméable.

6. Modèle multicellulaire selon la revendication 5, dans lequel ledit insert poreux est choisi parmi une matrice de collagène comprenant optionnellement des glycosaminoglycanes et/ou des fibroblastes, un gel ou une membrane d'acide hyaluronique et/ou de collagène et/ou de fibronectine et/ou de fibrine, une membrane semi-perméable de nitrocellulose, de nylon^{®}, de téflon^{®}, de polycarbonate ou de polyéthylène ou de polypropylène ou de polyethylène térephtalate (PET), une membrane inorganique Anopore^{®} semi-perméable, une membrane d'acétate de cellulose, une membrane semi-perméable Biopore-CM^{®}, une membrane semi-perméable de polyester, une membrane d'acide polyglycolique.

7. Modèle multicellulaire selon l'une quelconque des revendications précédentes, dans lequel lesdits premier et second tapis cellulaires sont agencés selon des plans parallèles.

8. Modèle multicellulaire selon la revendication précédente, dans lequel le premier et le second tapis cellulaire sont agencés selon des plans parallèles horizontaux.

9. Modèle multicellulaire selon la revendication précédente, dans lequel le second tapis cellulaire figure un plan supérieur.

10. Modèle multicellulaire selon la revendication précédente, dans lequel ledit plan supérieur est formé par un fond d'un insert, dans lequel est disposé ledit second tapis cellulaire.

11. Modèle multicellulaire selon la revendication précédente, dans lequel ledit fond de l'insert présente une porosité convenant au développement de terminaisons nerveuses à l'extérieur dudit fond.

12. Modèle multicellulaire selon l'une quelconque des revendications précédentes, dans lequel ledit premier tapis cellulaire est disposé sur un support choisi parmi une matrice de collagène comprenant éventuellement des fibroblastes, un demie désépidermisé, un équivalent de derme, une membrane d'acide hyaluronique et/ou de collagène et/ou de fibronectine et/ou de fibrine, et un support inerte.

13. Modèle multicellulaire selon l'une quelconque des revendications précédentes, comprenant, en outre, au moins un type cellulaire additionnel choisi parmi des cellules endothéliales, des cellules du système immunitaire telles que des cellules de Langerhans, des lymphocytes T, des cellules dendritiques, des macrophages, ou encore des adipocytes.

14. Utilisation d'un modèle multicellulaire tel que défini selon l'une quelconque des revendications 1 à 13 pour cribler des agents susceptibles de moduler une manifestation d'un échange chimique susceptible d'intervenir entre des cellules nerveuses, des kératinocytes et/ou des mélanocytes.

15. Utilisation d'un modèle multicellulaire tel que défini selon l'une quelconque des revendications 1 à 13 pour identifier au moins une molécule susceptible d'être impliquée dans une manifestation d'un échange chimique susceptible d'intervenir entre des cellules nerveuses, des kératinocytes et/ou des mélanocytes.

16. Utilisation selon la revendication précédente, dans laquelle ledit échange chimique a un effet modulateur à l'égard de la mélanogenèse.

17. Procédé de criblage d'agents susceptibles de moduler la mélanogenèse, comprenant au moins les étapes consistant à :
a) disposer d'un modèle multicellulaire tel que défini selon l'une quelconque des revendications 1 à 13,
b) mettre en présence du modèle au moins un agent à cribler, et
c) déterminer la quantité de mélanine produite par ledit modèle, à l'issue de l'étape b).

18. Procédé selon la revendication précédente, dans lequel la quantité de mélanine produite à l'issue de l'étape c) est comparée à une quantité de mélanine produite par ledit modèle en absence d'agent à cribler.

19. Procédé selon la revendication 17 ou 18, dans lequel la mélanogenèse est stimulée par mise en oeuvre à l'étape a) d'un agent activant ou inhibant un type de cellule.

20. Procédé selon la revendication précédente, dans lequel ledit agent stimule les cellules nerveuses.

21. Procédé selon la revendication 19 ou 20, dans lequel ledit agent est choisi parmi des agonistes adrénergiques.

22. Procédé selon l'une quelconque des revendications 17 à 21, dans lequel l'agent à cribler est susceptible de moduler la mélanogenèse par action au niveau des cellules nerveuses et/ou des mélanocytes et/ou des kératinocytes.

## Claims

1. Multicellular model comprising at least, as cell type:
- keratinocytes,
- melanocytes, and
- nerve cells,
in which the keratinocytes and the melanocytes form a first cell layer, said nerve cells form a second cell layer devoid of physical contact with the first cell layer,
said first and second cell layers being arranged so as to be compatible with a manifestation of at least one cellular chemical exchange.

2. Multicellular model according to the preceding claim, in which the cellular chemical exchange has a modulatory effect with regard to a biological activity of at least one cell type.

3. Multicellular model according to the preceding claim, in which the biological activity is melanogenesis.

4. Multicellular model according to any one of the preceding claims, in which said first and second cell layers are arranged so as to divide a single chamber into at least two compartments.

5. Multicellular model according to any one of the preceding claims, in which the first and/or the second cell layer(s) is (are) placed on and/or in a porous or semi-permeable insert.

6. Multicellular model according to Claim 5, in which said porous insert is chosen from a collagen matrix optionally comprising glycosaminoglycans and/or fibroblasts, a gel or a membrane of hyaluronic acid and/or of collagen and/or of fibronectin and/or of fibrin, a semi-permeable membrane of nitrocellulose, of nylon^{®}, of Teflon^{®}, of polycarbonate, of polyethylene, of polypropylene or of polyethylene terephthalate (PET), a semi-permeable Anopore^{®} inorganic membrane, a cellulose acetate membrane, a Biopore-CM^{®} semi-permeable membrane, a semi-permeable polyester membrane, and a polyglycolic acid membrane.

7. Multicellular model according to any one of the preceding claims, in which said first and second cell layers are arranged along parallel planes.

8. Multicellular model according to the preceding claim, in which the first and the second cell layer are arranged along horizontal parallel planes.

9. Multicellular model according to the preceding claim, in which the second cell layer represents an upper plane.

10. Multicellular model according to the preceding claim, in which said upper plane is formed by a bottom of an insert, in which said second cell layer is placed.

11. Multicellular model according to the preceding claim, in which said bottom of the insert has a porosity suitable for the development of nerve endings outside said bottom.

12. Multicellular model according to any one of the preceding claims, in which said first cell layer is placed on a support chosen from a collagen matrix optionally comprising fibroblasts, a de-epidermalized dermis, a dermis equivalent, a hyaluronic acid and/or collagen and/or fibronectin and/or fibrin membrane, and an inert support.

13. Multicellular model according to any one of the preceding claims, also comprising at least one additional cell type chosen from endothelial cells, immune system cells such as Langerhans cells, T lymphocytes, dendritic cells or macrophages, or else adipocytes.

14. Use of a multicellular model as defined in any one of Claims 1 to 13, for screening for agents capable of modulating a manifestation of a chemical exchange that may take place between nerve cells, keratinocytes and/or melanocytes.

15. Use of a multicellular model as defined in any one of Claims 1 to 13, for identifying at least one molecule that may be involved in a manifestation of a chemical exchange that may take place between nerve cells, keratinocytes and/or melanocytes.

16. Use according to the preceding claim, in which said chemical exchange has a modulatory effect with regard to melanogenesis.

17. Method for screening for agents capable of modulating melanogenesis, comprising at least the steps consisting in:
a) providing a multicellular model as defined in any one of Claims 1 to 13,
b) placing the model in the presence of at least one agent to be screened, and
c) determining the amount of melanin produced by said model, at the end of step b).

18. Method according to the preceding claim, in which the amount of melanin produced at the end of step c) is compared with an amount of melanin produced by said model in the absence of agent to be screened.

19. Method according to Claim 17 or 18, in which the melanogenesis is stimulated by using, in step a), an agent that activates or inhibits a cell type.

20. Method according to the preceding claim, in which said agent stimulates the nerve cells.

21. Method according to Claim 19 or 20, in which said agent is chosen from adrenergic agonists.

22. Method according to any one of Claims 17 to 21, in which the agent to be screened is capable of modulating melanogenesis by acting on the nerve cells and/or melanocytes and/or keratinocytes.

## Patentansprüche

1. Mehrzelliges Modell, das, als Typ von Zellen, mindestens folgendes einschließt:
- Keratinozyten,
- Melanozyten, und
- Nervenzellen,
wobei die Keratinozyten und die Melanozyten einen ersten Zellteppich bilden, wobei die Nervenzellen einen zweiten Zellteppich, ohne physischen Kontakt mit dem ersten Zellteppich, bilden,
wobei der erste und der zweite Zellteppich so ausgestaltet sind, dass sie mit einem Auftreten von mindestens einem chemischen Zellstoffwechsel kompatibel sind.

2. Mehrzelliges Modell nach dem vorhergehenden Anspruch, wobei der chemische Zellstoffwechsel eine modulatorische Wirkung im Hinblick auf eine biologische Aktivität von mindestens einem Zelltyp aufweist.

3. Mehrzelliges Modell nach dem vorhergehenden Anspruch, wobei die biologische Aktivität die Melanogenese ist.

4. Mehrzelliges Modell nach einem der vorhergehenden Ansprüche, wobei der erste und der zweite Zellteppich so ausgestaltet sind, dass sie einen einzelnen abgeschlossenen Raum in mindestens zwei Kompartimente teilen.

5. Mehrzelliges Modell nach einem der vorhergehenden Ansprüche, wobei der erste und/oder der zweite Zellteppich auf und/oder in einem porösen oder halbdurchlässigen Einsatz angeordnet ist/sind.

6. Mehrzelliges Modell nach Anspruch 5, wobei der poröse Einsatz aus einer Matrix von Kollagen ausgewählt ist, umfassend gegebenenfalls Glycosaminoglycane und/oder Fibroblasten, ein Gel oder eine Membran von Hyaluronsäure und/oder von Kollagen und/oder von Fibronectin und/oder von Fibrin, eine halbdurchlässige Membran von Nitrocellulose, von Nylon^{®}, von Teflon^{®}, von Polycarbonat oder von Polyethylen oder von Polypropylen oder von Polyethylenterephthalat (PET), eine anorganische halbdurchlässige Anopore^{®}-Membran, eine Membran von Celluloseacetat, eine halbdurchlässige Biopore-CM^{®}-Membran, eine halbdurchlässige Membran von Polyester, eine Membran von Polyglycolsäure.

7. Mehrzelliges Modell nach einem der vorhergehenden Ansprüche, wobei der erste und der zweite Zellteppich in parallelen Ebenen ausgestaltet sind.

8. Mehrzelliges Modell nach dem vorhergehenden Anspruch, wobei der erste und der zweite Zellteppich in horizontalen paralleler Ebenen ausgestaltet sind.

9. Mehrzelliges Modell nach dem vorhergehenden Anspruch, wobei der zweite Zellteppich eine obere Ebene darstellt.

10. Mehrzelliges Modell nach dem vorhergehenden Anspruch, wobei die obere Ebene von einem Boden eines Einsatzes gebildet wird, in dem der zweite Zellteppich angeordnet ist.

11. Mehrzelliges Modell nach dem vorhergehenden Anspruch, wobei der Boden des Einsatzes eine Porosität aufweist, die zur Entwicklung von Nervenendigungen aus dem Boden heraus geeignet ist.

12. Mehrzelliges Modell nach einem der vorhergehenden Ansprüche, wobei der erste Zellteppich auf einem Träger angeordnet ist, der aus einer Matrix von Collagen ausgewählt ist, umfassend gegebenenfalls Fibroblasten, eine desepidermisierte Derma, ein Derma-Äquivalent, eine Membran von Hyaluronsäure und/oder von Kollagen und/oder von Fibronectin und/oder von Fibrin, und einen inerten Träger.

13. Mehrzelliges Modell nach einem der vorhergehenden Ansprüche, umfassend weiterhin mindestens einen zusätzlichen Zelltyp, ausgewählt aus Endothelzellen, Zellen des Immunsystems, wie Langerhans-Zellen, T Lymphozyten, dendritische Zellen, Macrophagen, oder auch Adipozyten.

14. Verwendung eines mehrzelligen Modells wie nach einem der Ansprüche 1 bis 13 definiert, zum Screening von Mitteln, die dazu ausgelegt sind, ein Auftreten eines chemischen Stoffwechsels, der zum Ablaufen in Nervenzellen, Keratinozyten und/oder Melanozyten geeignet ist, zu modulieren.

15. Verwendung eines mehrzelligen Modells wie nach einem der Ansprüche 1 bis 13 definiert, zur Identifizierung von mindestens einem Molekül, das dazu ausgelegt ist, an einem Auftreten eines chemischen Stoffwechsels, der zum Ablaufen in Nervenzellen, Keratinozyten und/oder Melanozyten geeignet ist, beteiligt zu sein.

16. Verwendung nach dem vorhergehenden Anspruch, wobei der chemische Stoffwechsel eine modulatorische Wirkung im Hinblick auf die Melanogenese besitzt.

17. Verfahren zum Screening von Mitteln, die dazu ausgelegt sind, die Melanogenese zu modulieren, umfassen mindestens die Schritte, die bestehen aus:
a) Vorlegen eines mehrzelligen Modells, wie nach einem der Ansprüche 1 bis 13 definiert,
b) In Kontakt Bringen des Modells mit mindestens einem zu screenenden Mittel, und
c) Bestimmen der von dem Modell am Ende von Schritt b) produzierten Menge von Melamin.

18. Verfahren nach dem vorhergehenden Anspruch, wobei die Menge von am Ende von Schritt c) produziertem Melamin mit einer Menge von Melamin verglichen wird, die von dem Modell in Abwesenheit von einem zu screenenden Mittel produziert wird.

19. Verfahren nach Anspruch 17 oder 18, wobei die Melanogenese durch die Verwendung bei Schritt a) von einem einen Typ von Zelle aktivierenden oder inhibierenden Mittel stimuliert wird.

20. Verfahren nach dem vorhergehenden Anspruch, wobei das Mittel die Nervenzellen stimuliert.

21. Verfahren nach Anspruch 19 oder 20, wobei das Mittel aus adrenergen Agonisten ausgewählt wird.

22. Verfahren nach einem der Ansprüche 17 bis 21, wobei das zu screenende Mittel dazu ausgelegt ist, die Melanogenese durch Wirkung an den Nervenzellen und/oder den Melanozyten und/oder den Keratozyten zu modulieren.
